# FASCICULE DE BREVET EUROPEEN

(11) **EP 1 127 561 B1**
(45) Date de publication et mention de la délivrance du brevet: **27.04.2005**
(21) Numéro de dépôt: 00400502.1
(22) Date de dépôt: 24.02.2000
(51) Int. Cl.: A61F 9/007

(54) **Appareil d'intubation lacrymale**
Vorrichtung zur Intubation des Tränenkanals
Device for intubation of lacrimal duct

(43) Date de publication de la demande: 29.08.2001
(73) Titulaire: Becker, Bruce B., Encino, CA 91316 (US)
(72) Inventeur: Becker, Bruce B., Encino, CA 91316 (US)
(74) Mandataire: Faber, Jean-Paul

(56) Documents cités:
- FR-A- 2 735 697
- FR-A- 2 741 538
- US-A- 5 318 513

## Description

La présente invention concerne un appareil d'intubation lacrymale en silicone et plus particulièrement un appareil asymétrique comprenant un segment de très grand diamètre qui est inséré de façon transnasale.

La glande lacrymale produit la couche aqueuse du film lacrymal. La partie orbitaire de la glande lacrymale est située dans l'orbite temporale supérieure. Les canalicules provenant de la glande lacrymale principale traversent la glande lacrymale palpébrale adjacente pour déboucher dans le cul-de-sac conjonctival supérieur qui est sur la face postérieure du haut de la paupière supérieure. Les glandes lacrymales accessoires dans les paupières supérieure et inférieure contribuent également à la production des larmes.

Les larmes imprègnent l'oeil et s'écoulent ensuite dans les points lacrymaux supérieur et inférieur, qui sont situés sur les bords des paupières supérieure et inférieure internes. Les larmes s'écoulent ensuite par les canalicules supérieur et inférieur, le canalicule commun, le sac lacrymal et descendent par le canal nasolacrymal dans le nez. Le canal nasolacrymal peut être bouché, de façon congénitale ou acquise à l'âge adulte. Si le canal nasolacrymal se bouche, les larmes ne peuvent plus s'écouler de la surface de l'oeil par le système lacrymal dans le nez. Les larmes s'accumulent alors sur les yeux et se répandent par dessus les paupières sur le visage. Le patient doit constamment se tamponner les yeux avec un mouchoir. De plus, les larmes stagnent dans le sac lacrymal, ce qui permet aux bactéries de se multiplier. Le sac lacrymal alors s'infecte (dacryocystite). Sous l'effet de la dacryocystite, le sac lacrymal devient enflé, rouge et douloureux. Du pus suinte depuis le sac lacrymal par le canalicule sur l'oeil. Ceci conduit à ce que des matières purulentes couvrent constamment l'oeil. A la longue, la dacryocystite ne répond pas aux antibiotiques et la chirurgie devient nécessaire.

La dacryocystorhinostomie (DCR) est l'opération chirurgicale utilisée pour corriger l'obstruction du canal nasolacrymal. Dans une DCR, une nouvelle ouverture (orifice) est formée entre le sac lacrymal et le nez. Celle-ci permet aux larmes de s'écouler depuis le sac lacrymal par l'orifice de DCR dans le nez. Une DCR ouverte ou par incision nécessite une incision sur le côté du nez. Dans une DCR ouverte, un grand orifice de DCR est créé en faisant une ouverture de 17 mm et plus dans la muqueuse et l'os. Cette procédure est associée à une morbidité significative, une longue guérison, et le risque de cicatrices et d'hémorragie. Par contre, une DCR endoscopique transnasale est associée à une morbidité beaucoup plus faible, pas d'incision, et une durée de guérison rapide. Une DCR endoscopique peut être effectuée à l'aide d'instruments de chirurgie lacrymale ou sinusale, un laser ou un cathéter à ballonnet. L'orifice de DCR endoscopique est plus petit (5 à 9 mm) que celui d'une DCR ouverte. Etant donné que l'orifice de DCR ne fait que 5 à 9 mm (0,1969 à 0,354 pouce) de diamètre, un extenseur est requis pour maintenir l'orifice de DCR ouvert après chirurgie. Sinon, l'inflammation et la cicatrisation post-opératoires peuvent provoquer sa fermeture.

Pour la sélection d'un appareil convenable, il est nécessaire de garder à l'esprit les dimensions et propriétés des canalicules, qui forment la partie la plus étroite du système lacrymal, ainsi que les propriétés du matériau de l'appareil de silicone et la technique d'insertion de l'appareil. Un patient adulte possède des canalicules qui font environ 0,5 mm (0,01969 pouce) de diamètre. Il est possible, cependant, d'utiliser des extenseurs en forme de tube de silicone qui sont quelque peu plus grands en terme de diamètre extérieur, parce qu'ils possèdent un certain degré d'élasticité, et parce que les appareils d'intubation lacrymale tubulaires de silicone tendent à devenir plus fins lorsqu'ils sont placés sous tension lorsqu'ils sont passés dans le système lacrymal. La taille de l'appareil d'intubation de silicone qui peut être utilisé atteint une limite supérieure pour l'appareil d'intubation de diamètre maximal qui peut être passé dans les canalicules facilement et sans endommager ceux-ci.

Le diamètre de l'appareil d'intubation lacrymale de diamètre maximal qui peut être utilisé ainsi est de 1,346 mm.

Il a été proposé dans l'art antérieur d'utiliser un appareil tubulaire de silicone possédant un diamètre uniforme de 0,51 mm . Cependant, ceci est un diamètre trop faible pour maintenir l'orifice de DCR endoscopique ouvert.

Un tube de plus grand diamètre possédant un diamètre de 0,94 mm a également été utilisé dans l'art antérieur. Ce tube de plus grand diamètre est encore trop petit et souple pour dilater l'orifice de DCR à ballonnet. De plus, le diamètre maximal provoque des irritations et des défauts épithéliaux sur la cornée et la conjonctive à l'endroit où le tube repose contre l'oeil dans le canthus interne. Il peut également obstruer l'écoulement par les canalicules étroits. Le tube de silicone comporte une sonde métallique rigide à chaque extrémité. Pour placer le tube dans le système lacrymal, la sonde est passée par le point lacrymal, le canalicule, le sac lacrymal et l'orifice de DCR dans le nez. La sonde est saisie dans le nez et descendue dans le nez et hors de la narine. La sonde tire le tube de silicone connecté par le point lacrymal, le canalicule, le sac lacrymal et l'orifice de DCR dans le nez. Les deux extrémités du tube sont alors coupées à 1½ cm à l'intérieur de la narine. Le tube est laissé en place pendant environ six mois.

Dans un effort pour dilater l'orifice de DCR endoscopique à l'aide d'un appareil d'intubation lacrymale de plus grand diamètre, qui est trop grand pour passer dans les canalicules, un appareil court, plus rigide, qui peut être placé depuis la fosse nasale a été proposé. L'appareil court de grand diamètre est poussé depuis l'intérieur du nez à travers l'orifice de DCR endoscopique dans le sac lacrymal. Une fois en place, l'appareil d'intubation lacrymale s'étend depuis le sac lacrymal, à travers l'orifice de DCR endoscopique, dans le nez. Un certain nombre de tels appareils ont été essayés. Ils posent plusieurs problèmes. Ils sont difficiles ou impossibles à pousser en place. Ils tombent fréquemment peu après la chirurgie. Des tentatives pour les fixer sur un tube de silicone ne sont que d'une aide minime. Ces appareils d'intubation lacrymale peuvent provoquer des inflammations, des infections et des cicatrices.

Le demandeur à conçu un tube de silicone "ventru" qui possède un grand diamètre dans la partie qui dilate un orifice de DCR à ballonnet. Un exemple est décrit dans le brevet US-A-6 113 567 publié le 5 septembre 2000. Il possède un faible diamètre dans le segment qui repose contre l'oeil. La partie de grand diamètre (généralement de 1,32 mm de diamètre) ne peut pas être passée par le point lacrymal et les canalicules de faible diamètre sans glisser de la sonde. par conséquent, chaque extrémité du tube comprend une longue partie de faible diamètre. Les extrémités de faible diamètre du tube sont fixées à la sonde. Une fois que la sonde est saisie dans le nez, elle est descendue dans le nez et l'extrémité de faible diamètre du tube liée passe par le point lacrymal, les canalicules, le sac lacrymal et l'orifice de DCR à ballonnet dans le nez. Ceci est effectué avec chaque extrémité du tube. Une fois en place, les extrémités du tube sont coupées à 1½ cm à l'intérieur de la narine. Le segment de plus grand diamètre du tube doit être situé dans les canalicules distaux, le sac lacrymal et l'orifice de DCR à ballonnet s'étendant dans le nez. L'extrémité de faible diamètre du tube doit être découpée.

Cependant, il existe deux problèmes avec ce tube "ventru". Chez certains patients, il peut être difficile de tirer les segments de grand diamètre à travers les canalicules. Si les canalicules sont quelque peu étroits, les tubes peuvent se rompre lorsque le chirurgien exerce une traction sur le segment distal étroit dans le nez en tentant de passer le gros segment par les canalicules dans le sac lacrymal. Un deuxième problème est que le passage des segments de grand diamètre dans les canalicules peut étirer suffisamment les canalicules pour les endommager et provoquer des fibroses (cicatrices). Dans ce cas, les canalicules deviennent sténosés (étroits) et obstruent l'écoulement des larmes une fois que le tube est retiré. Ceci provoque des larmoiements. De plus, un tube d'un diamètre de plus de 1,35 mm est nécessaire pour dilater l'orifice de DCR à ballonnet dans certains cas. Cependant, comme il a été expliqué, il est impossible de faire passer un tube d'un diamètre beaucoup plus grand que 1,35 mm par les canalicules dans le sac lacrymal.

Si un tube de silicone peut être passé dans le canalicule depuis la cavité nasale, un tube d'un diamètre beaucoup plus grand peut être utilisé. Cependant, pendant la chirurgie, il n'est généralement pas possible de voir le canalicule commun depuis l'intérieur du nez, même visualisé à l'aide d'un endoscope. Il n'est certainement pas possible d'enfiler un tube de silicone depuis le nez dans le petit canalicule commun. Pour cette raison, l'intubation de silicone depuis le nez jusqu'au sac lacrymal par les canalicules n'a jamais été effectuée.

Une proposition initiale d'insérer une grande canule dans le système lacrymal est décrite dans le brevet U.S. n° 2 154 968. Ce brevet propose l'utilisation d'une canule en spirale en fil métallique fin, dont le diamètre externe peut varier jusqu'à 5/32 pouce. La spirale est effilée à une extrémité pour faciliter l'insertion dans le conduit lacrymal. Un tube est inséré à travers le point lacrymal, le canalicule, le sac lacrymal et le canal nasolacrymal dans la fosse nasale, et un fil est enfilé dans le tube. L'extrémité du fil est tirée hors de la narine et la canule en spirale est enfilée sur le fil avec son extrémité effilée tournée vers le haut. Une enveloppe de support est placée sur le fil et la canule est remontée dans le canal nasolacrymal depuis la fosse nasale. Cependant, la canule du brevet 2 154 968 n'est pas un appareil d'intubation lacrymale, c'est un conduit.

Cette canule est constituée d'un métal rigide, non de silicone. La canule n'est pas placée dans un orifice chirurgical formé pour drainer les larmes dans la fosse nasale. En fait, la proposition n'est pas cliniquement viable. Elle conduirait à des infections et des lésions chroniques et le canal se refermerait après le retrait de la canule en spirale. De plus, la procédure proposée dans le brevet 2 154 968 serait très difficile à effectuer pour un chirurgien. Par exemple, le chirurgien ne serait pas en mesure de voir si la canule est correctement positionnée, parce que la visualisation du canal nasolacrymal est bouchée par le cornet inférieur.

Le brevet U.S. n° 5 437 625 décrit un dispositif sous la forme d'un seul tube de silicone flexible comprenant un segment central souple et plus fin et une paire de segments terminaux de plus grand diamètre avec des extrémités libres qui sont pointues et hermétiques. Les segments terminaux comportent des petites coupures qui reçoivent des sondes métalliques s'étendant dans les extrémités fermées au cours de l'intubation. Il a été observé que les extrémités pointues des segments terminaux, étant rigides et dures, sont traumatisantes et sont trop dommageables pour les canalicules.

Le brevet US-A-4 305 395 décrit l'utilisation de sondes métalliques insérées dans des ouvertures dans les parois latérales de gaines tubulaires en polyamide fixées aux extrémités d'une longueur de tubulure en caoutchouc silicone. Les extrémités des sondes sont en butée contre les extrémités distales fermées des gaines tubulaires. La tubulure est ensuite positionnée dans le système lacrymal en passant les sondes insérées dans les gaines dans le système lacrymal par dessus le nez. Les sondes sont retirées, et les gaines sont saisies et tirées pour positionner la tubulure dans le système lacrymal.

Le brevet US-A-4 380 239 est un autre exemple de l'utilisation de sondes pour l'intubation d'un tube en caoutchouc silicone. Les sondes, qui sont formées de fil d'acier, sont insérées dans l'extrémité ouverte du tube pour faciliter l'insertion du tube dans un canalicule dans le conduit lacrymal.

Dans les méthodes des brevets 4 305 395 et 4 380 239, les gaines ou sondes doivent être tirées depuis la narine et, pendant la procédure, des épistaxies et des fractures osseuses du cornet inférieur se produisent parfois.

Le document US-A-5 318 513 décrit un appareil d'intubation lacrymale en silicone présentant des segments de différentes sections et un élément gonflable à une extrémité.

Les appareils d'intubation lacrymale de silicone de l'art antérieur sont tubulaires. Etant donné qu'un tube ne peut être fabriqué que par un procédé de moulage lorsque le tube entier est d'un diamètre uniforme, un tube de diamètre variable ne peut être fabriqué que par un procédé d'extrusion. Le procédé d'extrusion, moins fiable, permet de fabriquer des tubes si la variation de diamètre est minime. Cependant, l'extrusion seule n'est pas un procédé réalisable si, comme dans la présente invention, il existe une forte variation du diamètre des segments de l'appareil. De plus, des appareils extrudés tubulaires possèdent une surface qui est plus rugueuse que la surface possible d'un appareil moulé. Le procédé d'extrusion est également coûteux, parce qu'un pourcentage significatif des tubes extrudés n'est pas conforme au diamètre spécifié et doit être rejeté.

L'appareil d'intubation lacrymale selon l'invention comprend les caractéristiques définies dans la revendication 1.

L'objet de l'invention est de proposer un nouveau type d'appareil d'intubation lacrymale destiné à être inséré dans un orifice formé par chirurgie entre le sac lacrymal et la fosse nasale du patient caractérisé en ce qu'il est constitué d'un élément allongé souple en silicone présentant, à une extrémité, un segment terminal ayant un diamètre plus important que les autres parties, ce segment terminal étant raccordé à un segment de diamètre moyen ayant un plus petit diamètre que celui du segment terminal par un segment central fin, tandis que l'extrémité libre du segment de diamètre moyen est prolongée par un segment distal ayant un diamètre inférieur à celui du segment de diamètre moyen et dont l'extrémité libre comporte une lumière.

De préférence, le segment terminal présente un diamètre de 1,905 mm et une longueur de 12 cm.

De préférence, le segment de diamètre moyen présente un diamètre de 1,143 mm et une longueur de 15 cm.

De préférence, le segment distal présente un diamètre de 0,762 mm et une longueur de 7 cm.

De préférence, le segment central fin présente un diamètre de 0,762 mm et une longueur de 20 mm.

De préférence, l'appareil comprend des segments de transition entre le segment terminal et le segment central fin, entre ce dernier et le segment de diamètre moyen et entre ce dernier et le segment distal.

Enfin, de préférence, le segment terminal comporte une lumière s'ouvrant à son extrémité libre.

L'invention va maintenant être décrite avec plus de détails en se référant à des modes de réalisation particuliers donnés à titre d'exemple seulement et représentés au dessins annexés.
La figure 1 est une vue d'un premier mode de réalisation d'un appareil d'intubation lacrymale de l'invention (non dessiné à l'échelle) ;
La figure 1A est une vue d'un deuxième mode de réalisation d'un appareil d'intubation lacrymale de l'invention (non dessiné à l'échelle) ;
La figure 2 est une vue d'une tige métallique et d'une gaine utilisées dans les méthodes d'insertion de l'appareil d'intubation lacrymale de la figure 1 ;
La figure 3 est une vue représentant la tige de la figure 2 insérée dans la gaine de la figure 2 ;
La figure 4 est une sonde métallique utilisée dans les méthodes d'insertion de l'appareil d'intubation lacrymale de l'invention ;
Les figures 5 à 11 représentent les étapes d'un premier mode de réalisation d'une méthode d'insertion de l'appareil d'intubation lacrymale de l'invention ;
La figure 11A est un agrandissement d'un détail de la figure 11 ;
Les figures 12 à 15 représentent des étapes supplémentaires de la méthode des figures 5 à 11 ;
Les figures 16 à 18 représentent des étapes d'un deuxième mode de réalisation d'une méthode d'insertion de l'appareil d'intubation lacrymale de l'invention ;
La figure 18A est un agrandissement d'un détail de la figure 18 ; et
Les figures 19 à 22 représentent des étapes supplémentaires des méthodes des figures 16 à 18.

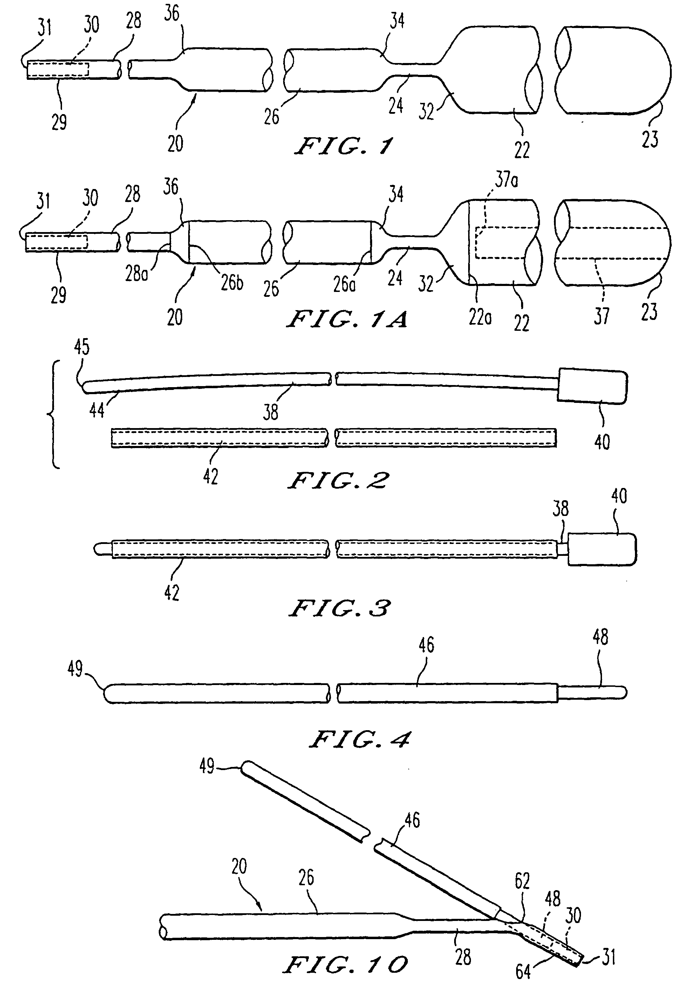

Un premier mode de réalisation d'un appareil d'intubation lacrymale de l'invention est représenté figure 1. L'appareil 20 est formé de caoutchouc silicone et comporte un segment de très grand diamètre 22 à une extrémité. L'expression "très grand diamètre" est utilisée pour signifier que le segment 22 possède un diamètre extérieur qui est supérieur au diamètre maximal des autres parties de l'appareil qui peuvent passer facilement dans les canalicules sans les endommager. Dans le cas d'un patient adulte type, les canalicules possèdent un diamètre intérieur d'environ 0,5 mm (0,01969 pouce) , et le diamètre maximal d'appareil qui peut être passé dans les canalicules facilement et sans endommager ceux-ci, est de 1,346 mm (0,053 pouce). En conséquence, le diamètre de segment de très grand diamètre 22 est supérieur à 1,346 mm. Dans un mode de réalisation préféré, le diamètre extérieur de segment de très grand diamètre 22 est de 1,905 mm (0,075 pouce); et le segment 22 fait 12 cm de long (4,724 pouces). L'extrémité 23 du segment 22 est doucement arrondie. Afin d'augmenter la flexibilité du segment 22, dans un deuxième mode de réalisation de l'appareil 20 représenté sur la figure 1A, une lumière 37 de 0,635 mm. de diamètre est formée, s'étendant de l'extrémité 23 à un point 37a à 5 mm de l'extrémité opposée du segment 22 ou sur toute la longueur du segment 22.

Dans les deux modes de réalisation représentés sur les figures 1 et 1A, l'autre extrémité du segment 22 est connectée à une extrémité d'un segment central relativement fin 24, qui possède un diamètre extérieur de 0,762 mm (0,030 pouce) et une longueur de 20 mm (0,787 pouce). Une fois que l' appareil est installé, un segment central fin 24 repose contre l'oeil dans le canthus interne et dans les canalicules. Le diamètre de 0,762 mm du segment 24 évite les irritations et défauts épithéliaux qui ont été associés aux segments de grand diamètre à cet emplacement et n'obstrue pas l'écoulement des larmes par les canalicules étroits.

L'autre extrémité du segment central 24 est connectée à une extrémité d'un segment de diamètre moyen 26, dont le diamètre extérieur peut être passé dans les canalicules du patient facilement et sans endommager les canalicules. Dans le mode de réalisation préféré, le diamètre extérieur du segment de diamètre moyen 26 est de 1,143 mm (0,045 pouce) et la longueur du segment 26 est de 15 cm (5,906 pouces).

L'autre extrémité du segment de diamètre moyen 26 est connectée à une extrémité du segment distal 28. Ce segment possède le même diamètre extérieur de 0,762 mm (0,030 pouce) que le segment central 24 et fait 7cm (2,756 pouces) de longueur, bien que le reste du segment distal 28 soit formé de silicone pleine, une lumière 30 est formée dans les 3 cm (1,181 pouces) distaux du segment distal 28. La lumière 30 fait donc 3 cm de long, s'étendant depuis l'extrémité distale 31 du segment distal 28, et est délimitée par une paroi latérale 29 et possède un diamètre intérieur de 0,3302 mm (0,013 pouce). Cependant la lumière 30 peut également s'étendre sur toute la longueur du segment distal 28.

Des zones de transition doucement effilées 32, 34 et 36, qui font 3 mm de longueur, relient respectivement le segment de très grand diamètre 22 au segment central 24, le segment central 24 au segment de diamètre moyen 26, et le segment de diamètre moyen 26 au segment distal 28.

Les appareils d'intubation lacrymale de l'art antérieur sont tubulaires et le premier mode de réalisation de l'appareil 20 représenté sur la figure 1 est de silicone pleine sauf la partie du segment distal 28 qui comporte lalumière 30. Dans le deuxième mode de réalisation, l'appareil est de silicone pleine sauf les parties du segment distal 28 et du segment de très grand diamètre 22 qui comportent les lumières 30 et 37, respectivement.

Etant donné la grande variation des diamètres extérieurs des segments de l'appareil 20, il n'est pas possible de former celui-ci par extrusion. L'appareil d'intubation lacrymale 20 de la figure 1 peut être moulé dans son intégralité. Ainsi, on réalise une fabrication plus fiable et économique de l'appareil et une meilleure conformité aux spécifications de conception. Un appareil moulé possède également une surface plus lisse que la surface d'un appareil extrudé tubulaire.

Cependant, l' appareil peut également être réalisé suivant une autre méthode qui consiste à ne mouler que la partie de l' appareil qui présente la plus grande variation de diamètre, les autres parties étant extrudées. En particulier, comme décrit sur la figure 1A, le segment central 24 et les segments de transition adjacents 32 et 34 sont moulés en une pièce, tandis que le segment de très grand diamètre 22, le segment de diamètre moyen 26, et le segment distal 28 sont extrudés en trois pièces supplémentaires. Les pièces sont alignées dans l'ordre approprié. Les extrémités du segment de très grand diamètre 22 et du segment de transition 32 sont en butée au niveau de la ligne 22a et sont contenues dans un moule qui met en prise de façon ajustée les extrémités en butée l'une avec l'autre. De façon similaire, les extrémités du segment de transition 34 et du segment de diamètre moyen 26 sont en butée au niveau de la ligne 26a et sont contenues dans un deuxième moule qui est en prise de façon ajustée avec les segments 34 et 26. De la silicone liquide est injectée dans le deuxième moule et est solidifiée pour souder les extrémités en butée l'une avec l'autre. L'autre extrémité 26b du segment de diamètre moyen 26 est alignée avec, mais espacée de l'extrémité 28a du segment distal 28. Un troisième moule met en prise de façon ajustée les extrémités adjacentes 26b et 28a des segments 26 et 28 et contient les extrémités 26b et 28a et l'espacement entre celles-ci. De la silicone liquide est injectée dans le moule, remplissant l'espacement, et se solidifie pour former un segment de transition 36 et souder les segments 26 et 28 l'un avec l'autre. Il est également possible d'omettre l'espacement, mettre en butée les extrémités 28a et 26b, et former un segment de transition 36 dans le troisième moule autour de l'extrémité du segment 28 lorsque les extrémités 28a et 26b sont soudées l'une avec l'autre. Bien que ces techniques résultent en des segments de transition 36 qui ne font qu'approcher la configuration doucement effilée des segments de transition 32 et 34, la forme du segment de transition 36 n'est pas critique et une surface inexacte sur le segment 36 ne doit pas interférer avec le fonctionnement de l' appareil 20.

Dans une autre méthode, le segment distal 28 et le segment de très grand diamètre 22 sont extrudés sous forme de tubes. Afin de former une lumière 30 dans le segment distal 28, une aiguille est insérée à l'emplacement de la lumière 30, et le reste du tube est rempli avec de la silicone liquide et laissée à solidifier. La lumière 37 dans le segment de très grand diamètre 22 est formée de la même façon. Si la lumière doit s'étendre sur toute la longueur du segment, cette étape est omise.

Le segment de diamètre moyen 26, qui est représenté comme étant plein sur la figure 1A, peut également être extrudé sous forme de tube. La lumière formée dans ce dernier peut s'étendre sur toute la longueur du segment, dont la lumière est fermée par la transition 34 à l'extrémité 26a et par la transition 36 à l'extrémité 26b.

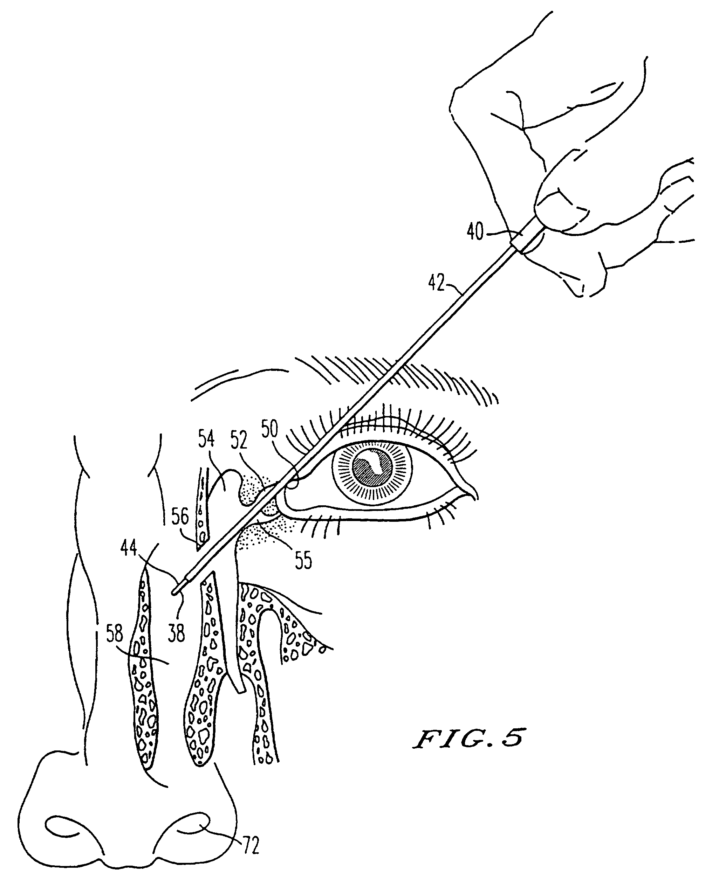

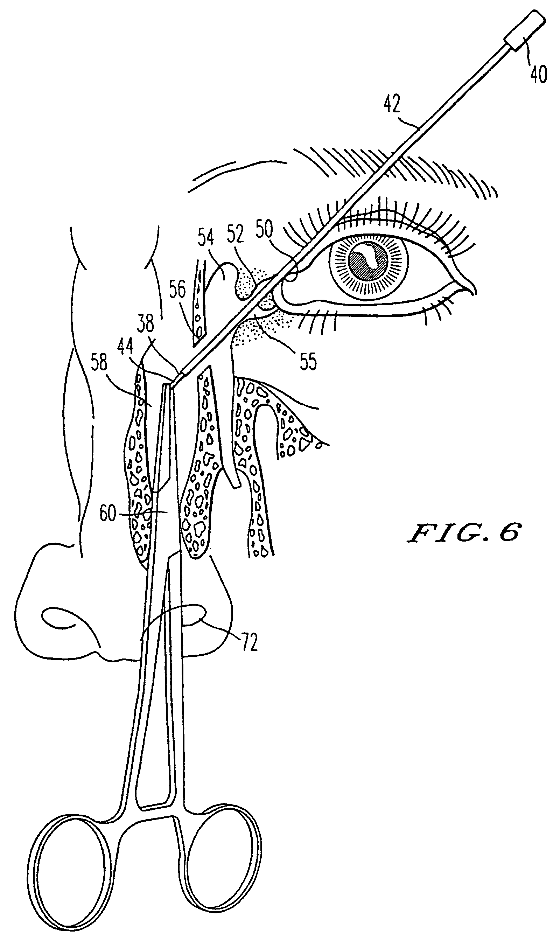

Les procédés d'insertion de l'appareil 20 dans le système lacrymal du patient nécessitent certains outils spéciaux. Comme décrit sur la figure 2, ceux-ci comprennent une tige métallique 38, comportant une tête 40, et une gaine en polyuréthane semi-rigide 42. La tige métallique 38 est formée avec une courbe doucement arquée et possède un diamètre extérieur d'environ 0,6604 mm (0,026 pouce). La tige 38 à partir de la base de la tête 40 fait 17,5 cm (6,89 pouces) de long. La tête 40 fait 1 cm de long et possède un diamètre de 1,016 mm (0,040 pouce). La gaine 42 fait 17 cm (6,693 pouces) de long et comporte une paroi très fine avec un diamètre intérieur de 0,889 mm (0,035 pouce). La tige 38 est insérée dans la gaine 42 comme décrit sur la figure 3. Lorsque la tête est en butée avec l'extrémité de la gaine 42, la section terminale distale 44 de la tige 38 s'étend hors de l'extrémité distale de la gaine 42 de 5 mm (0,1969 pouce) et comporte une pointe émoussée légèrement arrondie douce 45.

Les méthodes d'insertion de l' appareil mettent également en oeuvre l'utilisation d'une sonde métallique 46 représentée sur la figure 4. Cette sonde possède un diamètre de 0,584 mm (0,023 pouce) et une partie distale 48 qui possède un diamètre de 0,381 mm (0,015 pouce) et une longueur de 10 mm (0,3937 pouce) . L'extrémité proximale 49 de la sonde 46 est arrondie, et la longueur totale de la sonde 46 est de 19 cm (7,48 pouces).

Après la formation d'un orifice de DCR entre le sac lacrymal et la fosse nasale du patient par endoscopie ou par incision, l'appareil 20 est amené en place par les méthodes suivantes.

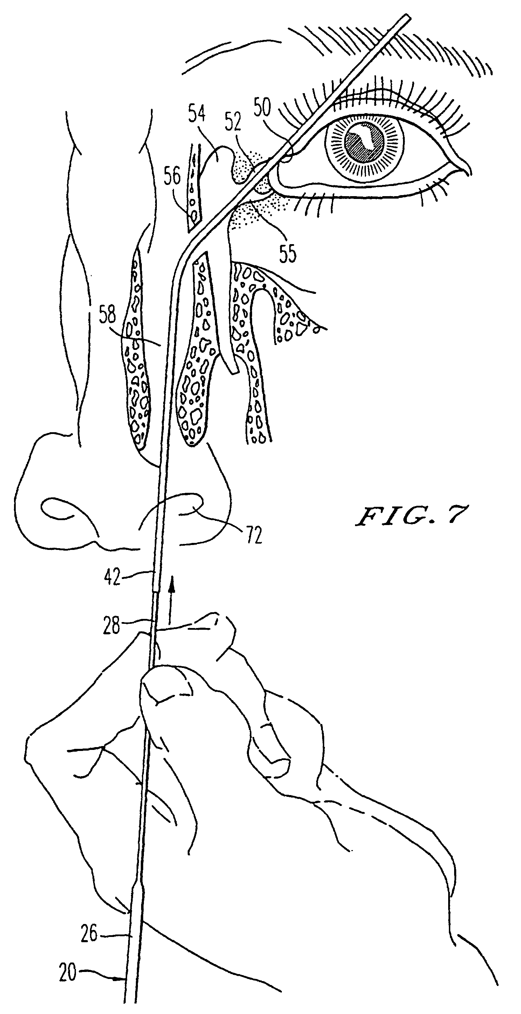

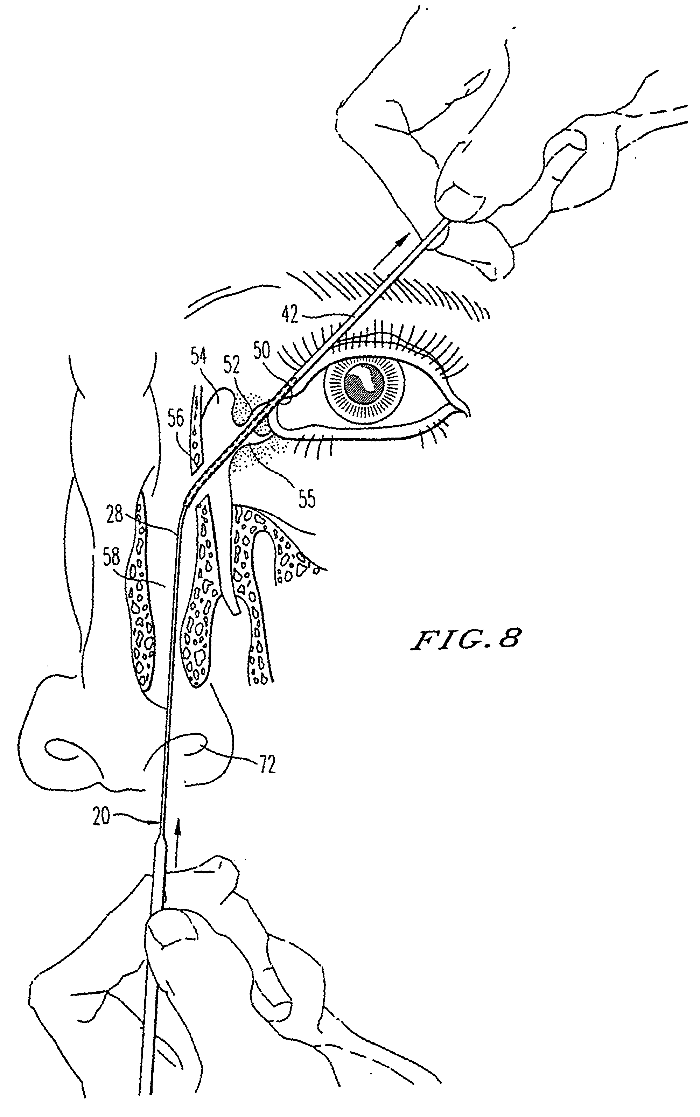

En référence à la figure 5, selon un premier mode de réalisation de la méthode, le chirurgien passe la tige 38 contenue dans la gaine 42 par le point lacrymal supérieur 50, le canalicule supérieur 52, le canalicule commun 55, le sac lacrymal 54 et l'orifice de DCR 56 du patient dans la fosse nasale 58 du patient. Comme décrit sur la figure 6, le chirurgien remonte dans le nez (fosse nasale 58) et saisit l'extrémité 44 de la tige métallique 38 avec un hémostat 60, en prenant soin de placer l'hémostat 60 autour de la section terminale distale 44 de la tige métallique 38 uniquement afin de ne pas endommager la gaine 42, et descend la tige du nez jusqu'à ce que la section terminale distale 44 de la tige 38 soit juste à l'extérieur de la narine 72 du patient. Le chirurgien tire ensuite dans le sens inverse la tige métallique hors de l'extrémité proximale de la gaine 42 et la retire.

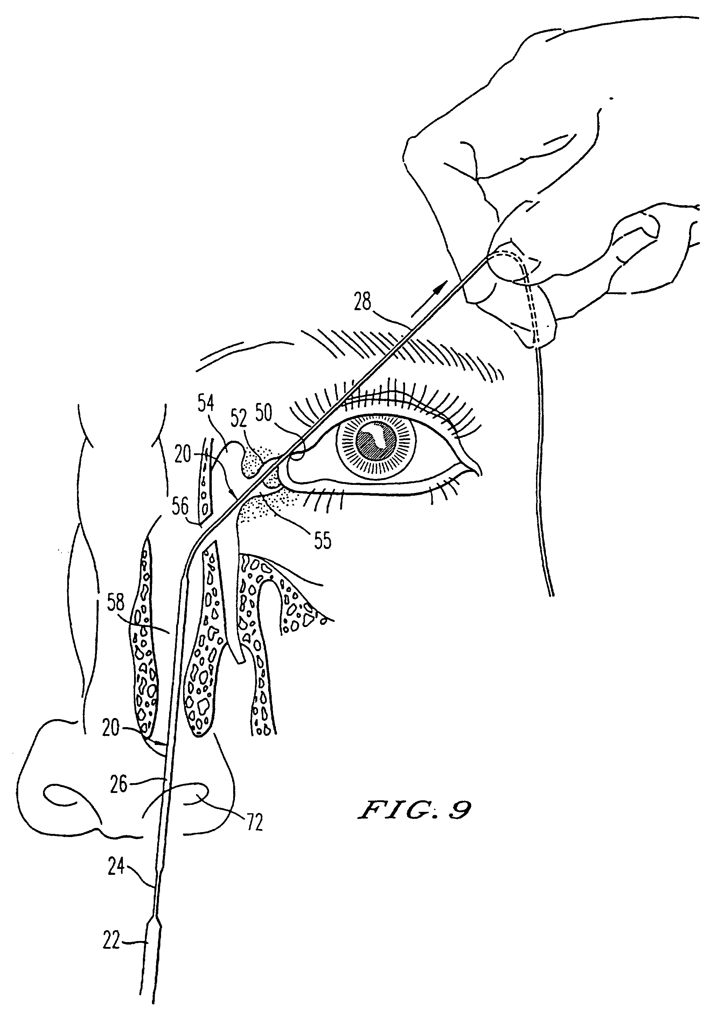

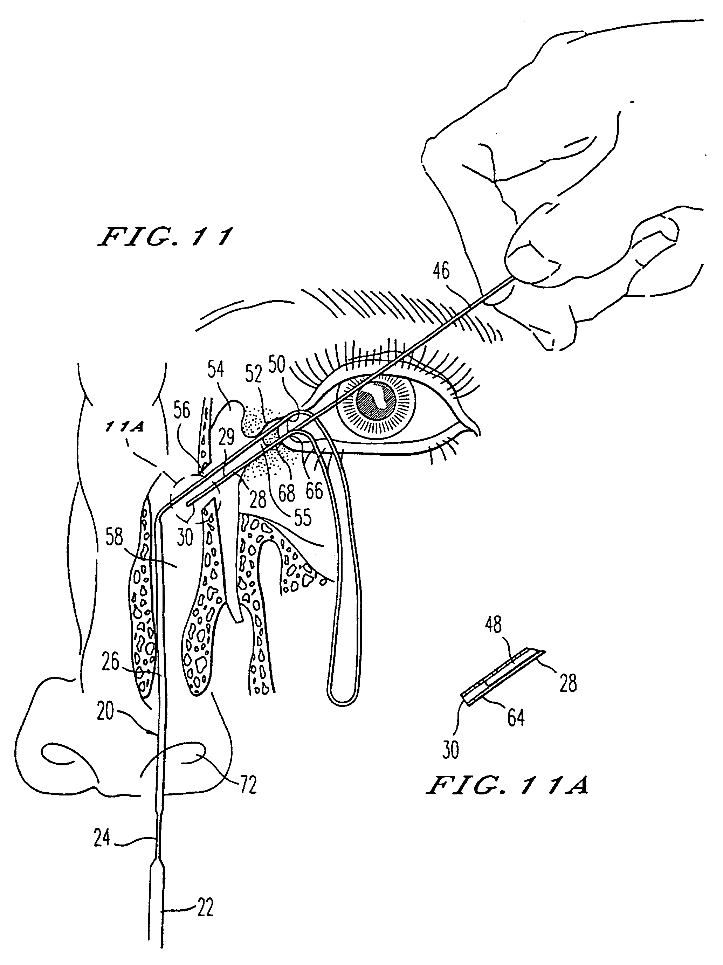

Comme décrit sur la figure 7, le chirurgien enfile ensuite l'extrémité distale 28 de l'appareil de silicone 20 dans l'extrémité distale de la gaine 42 de plusieurs centimètres. La figure 8 montre comment le chirurgien, en saisissant la gaine 42 d'une main et l'appareil 20 de l'autre main, tire ensuite la gaine 42 en remontant la fosse nasale 58 et hors du canalicule supérieur 52 et du point lacrymal supérieur 50. Cette manoeuvre fait passer le segment distal 28 de l'appareil 20 par l'orifice de DCR 56, le sac lacrymal 54, le canalicule commun 55, le canalicule supérieur 52, et le point lacrymal supérieur 50. Le chirurgien retire ensuite la gaine 42 du segment distal 28 et, comme décrit sur la figure 9, saisit le segment distal 28 et retire plus avant l'appareil 20 du canalicule 52 et du point lacrymal 50.

Comme décrit sur la figure 10, le chirurgien plie ensuite le segment distal 28 de l'appareil 20 et, en tenant la sonde 46, pique à travers la paroi latérale 28 de la lumière 30 du segment distal 28 avec la partie distale 48 de la sonde 46 pour former une ouverture 62 en un point à 10 mm (0,3937 pouce) de l'extrémité distale 31 du segment distal 28. La partie distale 48 de la sonde 46 est ensuite passée dans l'ouverture 62 jusqu'à ce que seulement 7 mm (0,2756 pouce) de partie de sonde 48 soit positionnée dans la lumière 30, laissant une section distale 64 de 3 mm (0,1181 pouce) de la lumière occupée par la sonde.

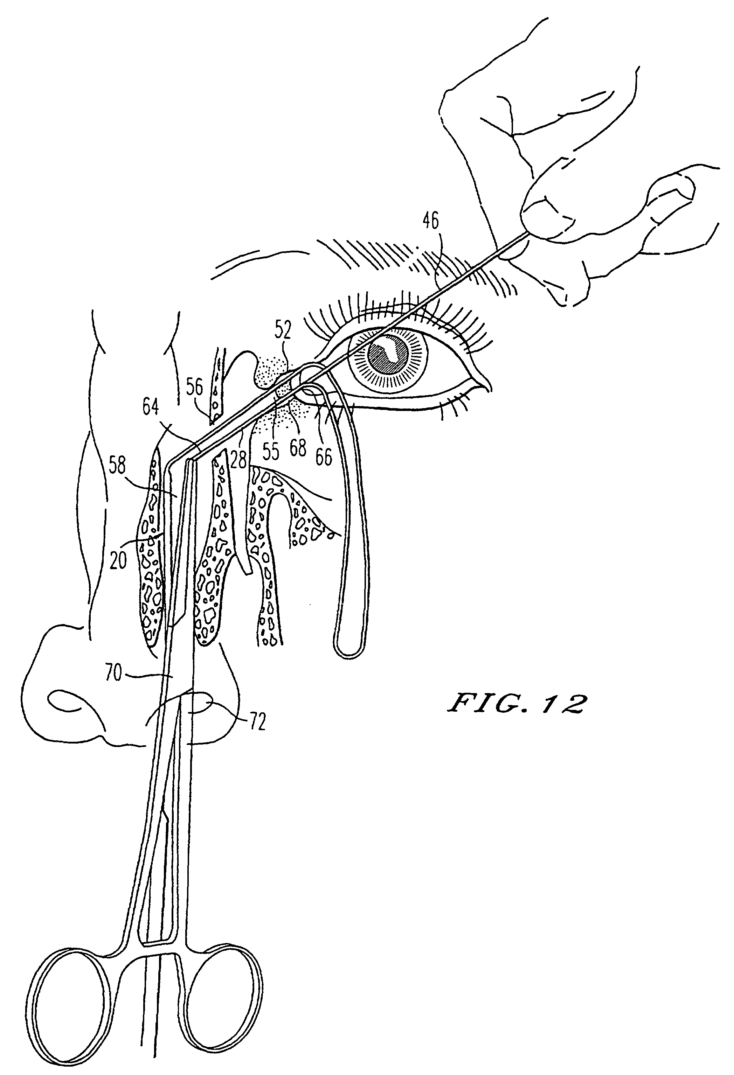

Comme décrit sur la figure 11 et le détail agrandi de la figure 11A, l'extrémité de la partie distale 48 de la sonde 46 étant insérée à travers la paroi latérale 29 dans la lumière 30, le chirurgien passe la sonde 46 dans le point lacrymal inférieur 66, le canalicule inférieur 68, le canalicule commun 55, le sac lacrymal 54 et l'orifice de DCR 56 dans la fosse nasale 58.

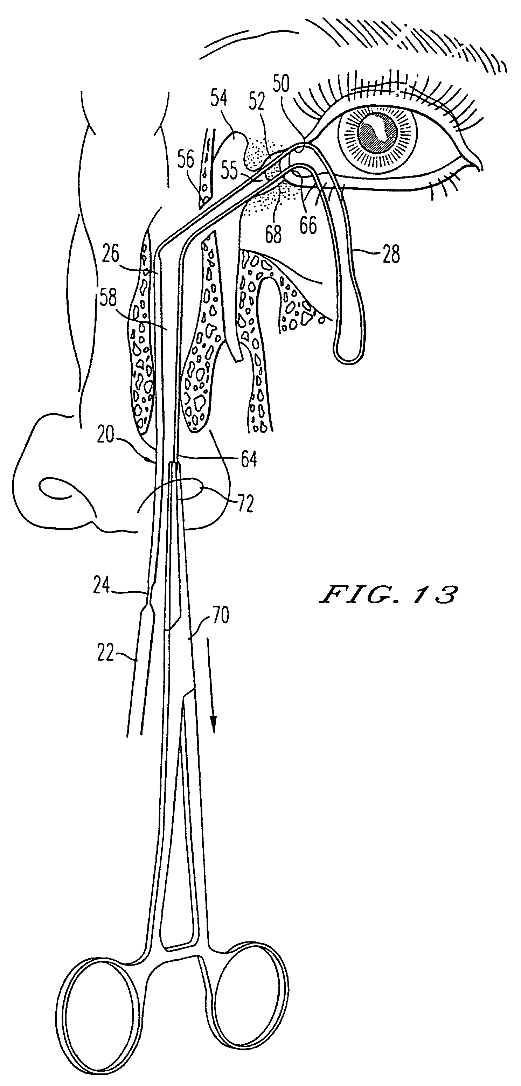

Le chirurgien remonte ensuite la fosse nasale 58 avec un hémostat 70 (voir figure 12) et saisit la section terminale 64 du segment distal 28 et descend l'appareil 20 dans la fosse nasale et hors de la narine 72. Parallèlement, le chirurgien remonte la sonde 46, hors du point lacrymal 66 pour la retirer de la lumière 30.

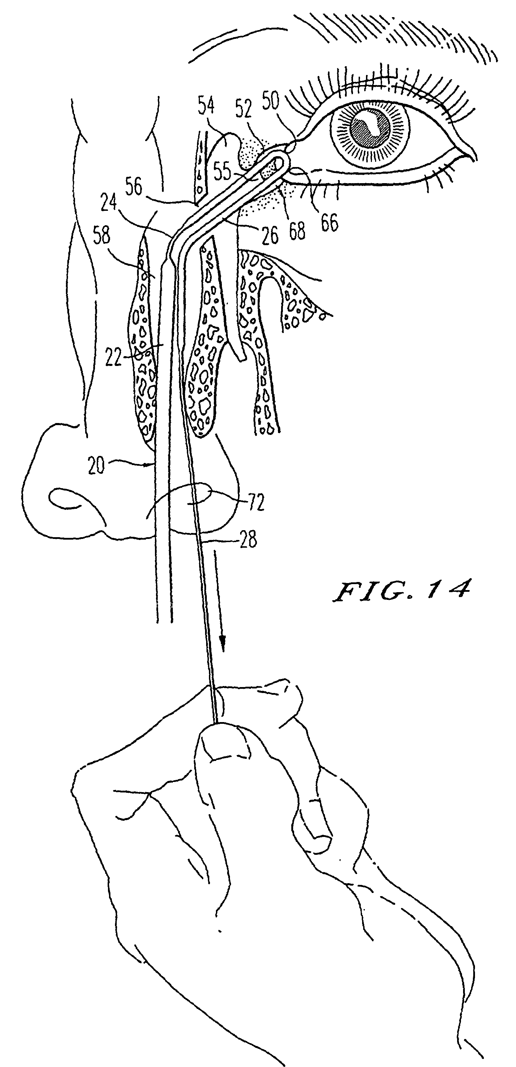

La figure 13 montre comment le chirurgien continue ensuite de tirer l' appareil 20 hors du nez. Pendant que le chirurgien continue de tirer, comme décrit sur la figure 14, il fait passer le segment de diamètre moyen 26 par le canalicule supérieur 52, le point lacrymal supérieur 50, le point lacrymal inférieur 66, le canalicule inférieur 68, le canalicule commun 55, le sac lacrymal 54 et l'orifice DCR 56 dans la fosse nasale 58.

Le chirurgien continue de tirer jusqu'à ce que le segment de très grand diamètre 22 soit amené en position dans l'orifice de DCR 56 avec une extrémité du segment 22 passée dans le sac lacrymal 54 jusqu'au niveau, mais pas dans l'extrémité côté sac lacrymal du canalicule commun 55 et l'extrémité opposée dans la fosse nasale 58. Le segment de diamètre moyen 26 est positionné dans l'orifice de DCR 56, une extrémité étant dans le sac lacrymal 54 adjacente à l'extrémité côté sac lacrymal du canalicule commun 55 et l'extrémité opposée dans la fosse nasale 58. Le segment central 24 est positionné de façon à occuper la fente palpébrale entre les points lacrymaux supérieur et inférieur 50 et 66 et dans les canalicules supérieur et inférieur 52 et 68 et le canalicule commun 55.

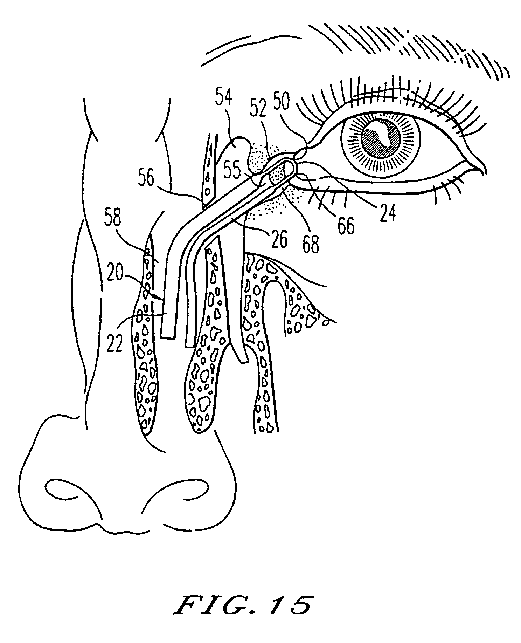

Une suture est utilisée pour nouer les extrémités des segments 22 et 26 dans la fosse nasale 58 l'une avec l'autre. Les deux extrémités dans la fosse nasale sont ensuite découpées d'environ 1 cm à l'intérieur de la narine 72. Ceci est la situation représentée sur la figure 15.

Cette méthode résout plusieurs problèmes. Elle permet au segment de très grand diamètre 22 de dilater l'orifice de DCR 56, malgré le fait qu'un segment d'un diamètre aussi grand ne peut pas être passé par les canalicules dans le sac lacrymal 54. Ceci est dû au fait que l'extrémité du segment distal de l' appareil 20 est passée par les canalicules depuis le côté nasal, rendant inutile pour le segment de très grand diamètre 22 d'un diamètre de 1,905 mm le passage par les canalicules de faible diamètre. Le segment de diamètre moyen 26 possédant un diamètre de 1,143 mm est le plus grand à passer par les canalicules, qu'il peut traverser facilement, réduisant au minimum le risque d'endommager les canalicules par étirement. Etant donné que le segment de diamètre moyen 26 passe dans les canalicules relativement aisément, il est improbable que le segment central 24 ou le segment distal 28 de 0,762 mm se casse étant donné que le segment 26 est passé dans les canalicules. Il doit être apprécié que la mise en oeuvre avec succès de la méthode est rendue possible par la configuration structurale et les dimensions particulières de l'appareil 20.

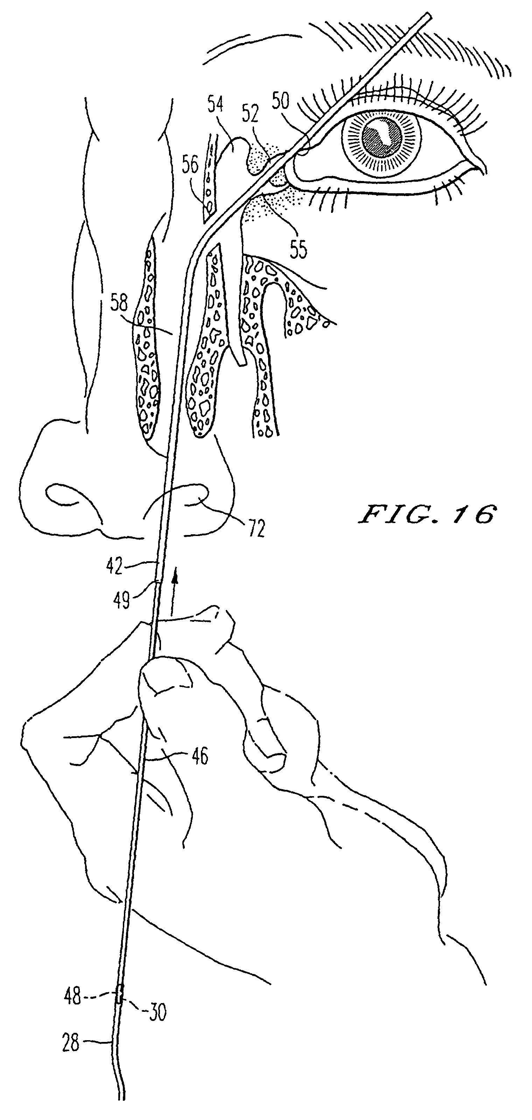

Un deuxième mode de réalisation alternatif d'une méthode d'insertion de l'appareil d'intubation lacrymale de l'invention est décrit ci-après en référence aux figures 16 à 23.

La méthode alternative commence de la même façon que la méthode des figures 5 à 15. Comme décrit sur la figure 5, le chirurgien passe la tige 38 contenue dans la gaine 42 dans le point lacrymal supérieur 50, le canalicule supérieur 52, le canalicule commun 55, le sac lacrymal 54 et l'orifice DCR 56 du patient dans la fosse nasale du patient 58. La tige métallique 38 est ensuite descendue, comme décrit sur la figure 6, jusqu'à ce que l'extrémité distale 44 de la tige 38 soit juste à l'extérieur de la narine du patient 72. Le chirurgien tire en sens inverse la tige métallique 38 hors de l'extrémité proximale de la gaine 42 pour la retirer, laissant la gaine en place.

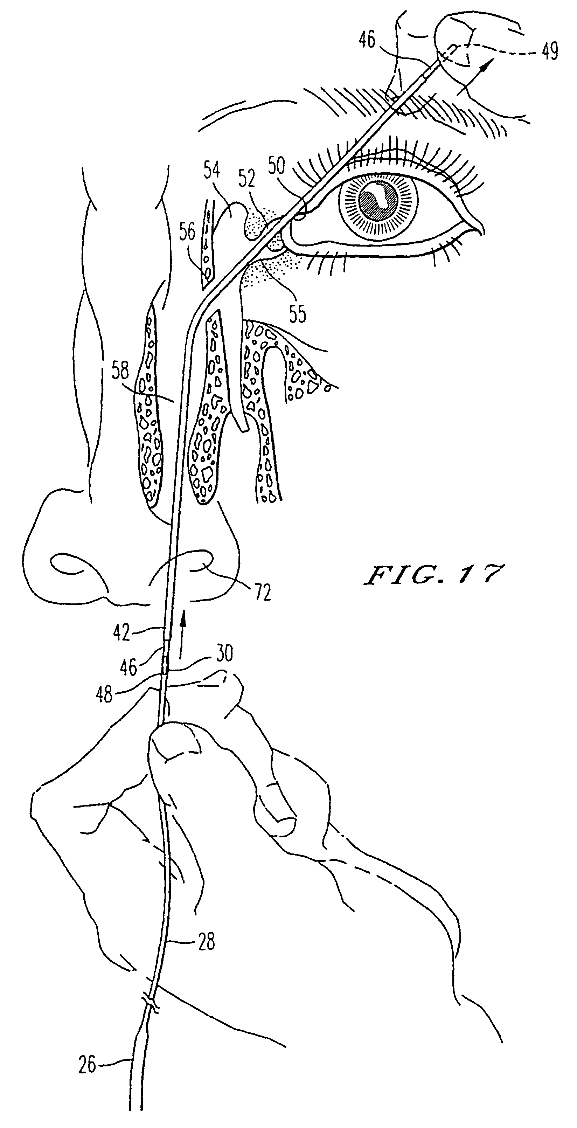

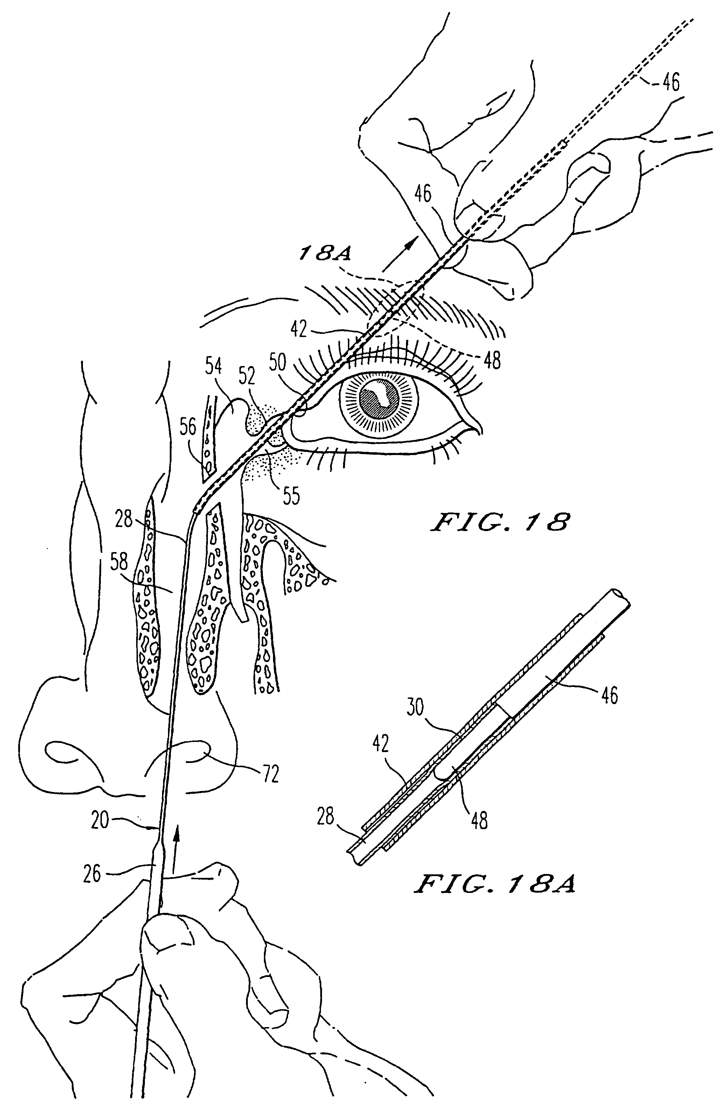

Une fois que la gaine est en place, selon la méthode alternative, la lumière distale 30 du segment distal 28 est sertie sur la partie distale fine 48 de la sonde métallique 46, comme décrit sur la figure 16. Comme décrit sur la figure 16, le chirurgien enfile l'extrémité 49 de la sonde 46 dans l'extrémité distale de la gaine 42 jusqu'à ce que l'extrémité 49 émerge de l'extrémité proximale de la gaine 46 comme décrit sur la figure 17. Le chirurgien saisit l'extrémité 49 de la sonde 46 et retire plus avant la sonde 46 de la gaine 42, tirant ainsi le segment distal 28 serti sur celle-ci de l'appareil 20 dans la gaine 42. Une fois que le segment distal 28 a été tiré suffisamment loin dans la gaine 42, le chirurgien, comme décrit sur la figure 18, saisit alors la gaine 42 d'une main et l'appareil 20 de l'autre main et remonte la gaine dans la fosse nasale 58 et hors du canalicule supérieur 52 et du point lacrymal supérieur 50. Cette manoeuvre fait passer la sonde 46 et le segment distal 28 par l'orifice de DCR 56, le sac lacrymal 54, le canalicule commun 55, le canalicule supérieur 52 et le point lacrymal supérieur 50. La figure 18A représente, dans une vue agrandie, le segment distal 28 serti sur une partie distale fine 48 de la sonde 46 dans la gaine 42.

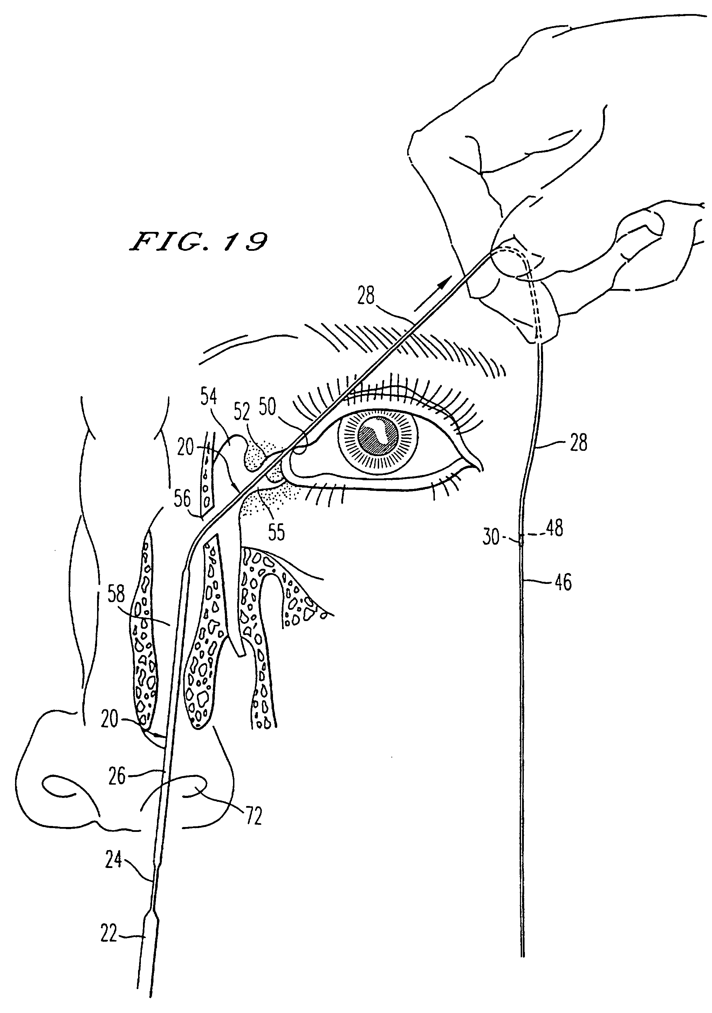

Le chirurgien retire ensuite la gaine 42 de la sonde 46 et du segment distal 28. Comme décrit sur la figure 19, le chirurgien saisit le segment distal 28 et retire plus avant l' appareil 20 du canalicule 52 et du point lacrymal 50.

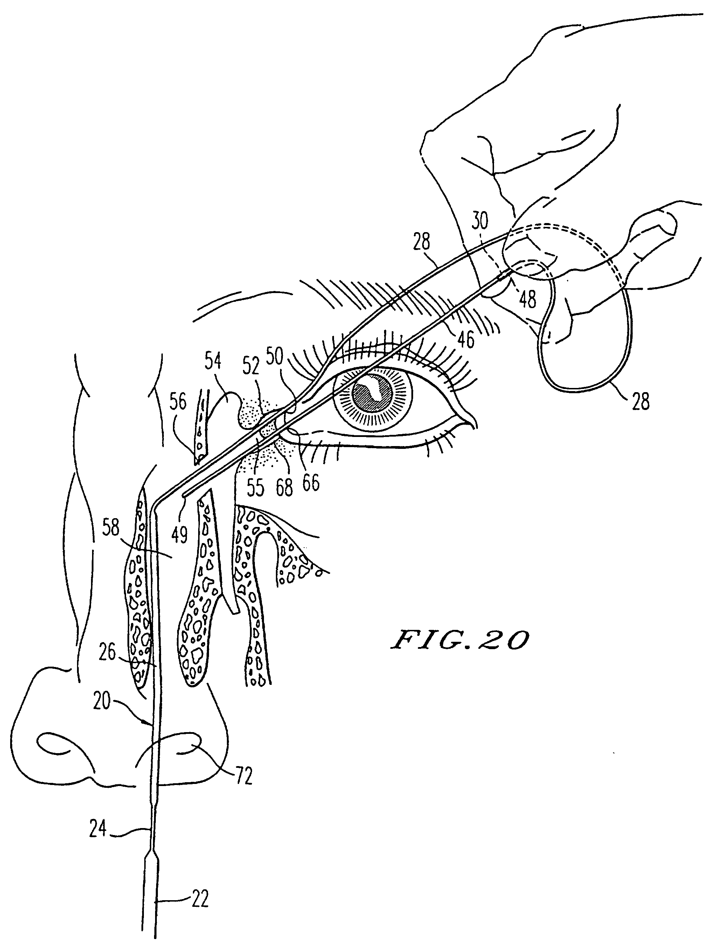

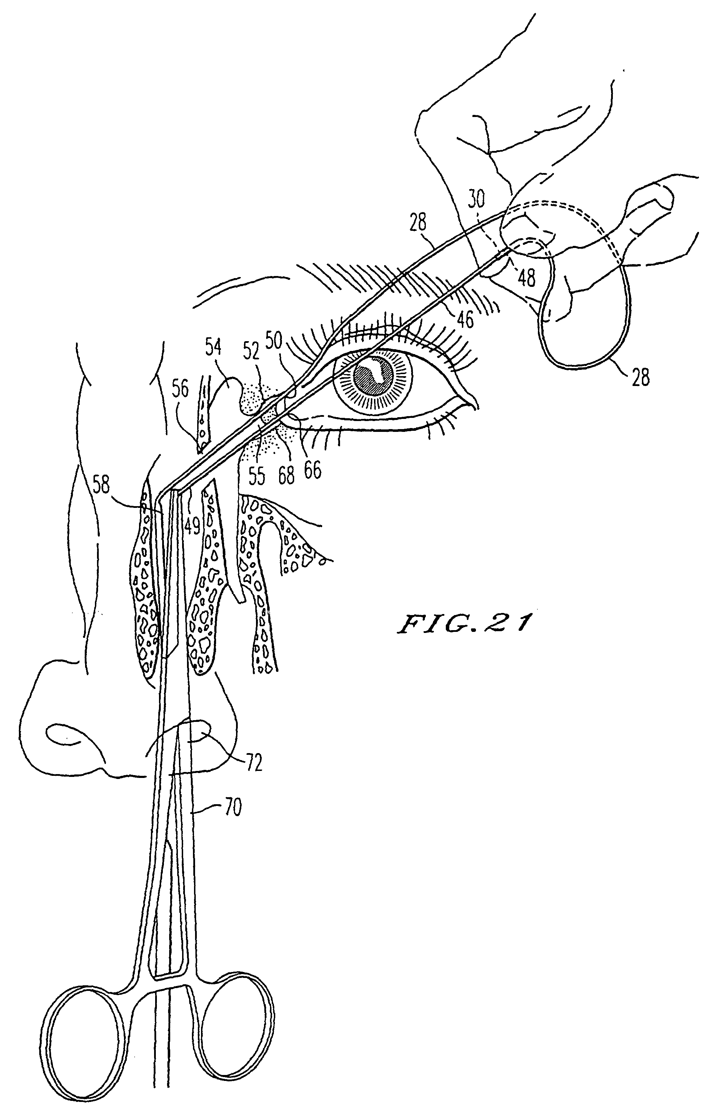

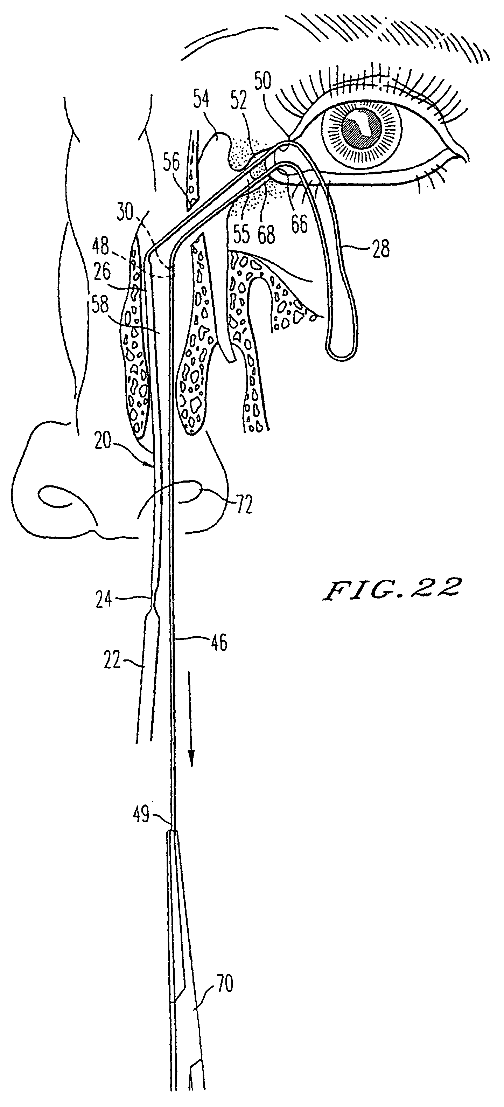

Comme décrit sur la figure 20, le chirurgien passe l'extrémité 49 de la sonde 46 par le point lacrymal inférieur 66, le canalicule inférieur 68, le canalicule commun 55, le sac lacrymal 54 et l'orifice de DCR 56 dans la fosse nasale. Le chirurgien remonte ensuite la fosse nasale 58 avec un hémostat 70 (voir figure 21) et saisit la sonde 46. Il descend ensuite la sonde 46 avec le segment distal 28 serti de l'appareil 20 depuis la fosse nasale 58 et hors de la narine 72 (voir figure 22).

A ce stade, le chirurgien retire la sonde 46 et tire sur le segment distal 28 (désormais libéré de la sonde 46), en la descendant plus avant depuis la fosse nasale 58 et hors de la narine 72, amenant ainsi le segment de diamètre moyen 26 par le canalicule supérieur 52, le point lacrymal supérieur 50, le point lacrymal inférieur 66, le canalicule inférieur 68, le canalicule commun 55, le sac lacrymal 54 et l'orifice de DCR 56 dans la fosse nasale 58. Ceci est la même position atteinte sur la figure 14 de la première méthode d'insertion de l'appareil. Les étapes restantes de la méthode sont les mêmes que celles décrites pour la première méthode comme décrit sur les figures 14 et 15.

On pourrait prévoir que l'appareil 20 peut être fabriqué avec le segment distal 28 déjà serti sur la partie 48 de la sonde 46.

Bien entendu, l'invention n'est pas limitée aux modes de réalisation qui viennent d'être décrits et représentés. On pourra y apporter de nombreuses modifications de détail, sans sortir pour cela du cadre de l'invention telle que définie dans les revendications 1 à 7.

## Revendications

1. Appareil d'intubation lacrymale destiné à être inséré dans un orifice formé par chirurgie entre le sac lacrymal et la fosse nasale du patient, ledit appareil étant constitué d'un élément allongé souple en silicone (20) présentant, à une extrémité, un segment terminal (22) ayant un diamètre plus important que les autres parties dudit appareil, ce segment terminal (22) étant raccordé à un segment (26) de diamètre moyen ayant un plus petit diamètre que celui du segment terminal par un segment central fin (24), tandis que l'extrémité libre du segment de diamètre moyen (26) est prolongée par un segment distal (28) ayant un diamètre inférieur à celui du segment de diamètre moyen (26) et dont l'extrémité libre comporte une lumière (31).

2. Appareil d'intubation lacrymale, selon la revendication 1, **caractérisé en ce que** le segment 5 terminal (22) présente un diamètre de 1,905 mm et une longueur de 12 cm.

3. Appareil d'intubation lacrymale, selon la revendication 1, **caractérisé en ce que** le segment de diamètre moyen (26) présente un diamètre de 1,143 mm et une longueur de 15 cm.

4. Appareil d'intubation lacrymale, selon la revendication 1, **caractérisé en ce que** le segment distal (28) présente un diamètre de 0,762 mm et une longueur de 7 cm.

5. Appareil d'intubation lacrymale, selon la revendication 1, **caractérisé en ce que** le segment central fin (24) présente un diamètre de 0,762 mm et une longueur de 20 mm.

6. Appareil d'intubation lacrymale, selon la revendication 1, **caractérisé en ce qu'**il comprend des segments de transition (32, 34, 36) entre le segment terminal (22) et le segment central fin (24), entre le segment central fin et le segment de diamètre moyen (26) et entre le segment de diamètre moyen et le segment distal (28).

7. Appareil d'intubation lacrymale, selon la revendication 1, **caractérisé en ce que** le segment terminal (22) comporte une lumière (37) s'ouvrant à son extrémité libre.

## Patentansprüche

1. Vorrichtung zur Intubation der Tränenwege für das Einbringen in eine mittels Chirurgie gebildete Öffnung zwischen dem Tränensack und der Nasengrube des Patienten, wobei die Vorrichtung aus einem biegsamen länglichen Element (20) aus Silikon besteht, das an einem Ende ein Endsegment (22) mit einem größeren Durchmesser als die anderen Teile der Vorrichtung besitzt, wobei das Endsegment (22) mit einem Segment (26) mit einem mittleren Durchmesser, das einen kleineren Durchmesser als das Endsegment hat, durch ein feines Zentralsegment (24) verbunden ist, während das freie Ende des Segments mit mittlerem Durchmesser (26) durch ein distales Segment (28) verlängert wird, das einen kleineren Durchmesser als das Segment mit mittlerem Durchmesser (26) hat und dessen freies Ende ein Licht (31) trägt.

2. Vorrichtung zur Intubation der Tränenwege nach Anspruch 1, **dadurch gekennzeichnet, dass** das Endsegment (22) einen Durchmesser von 1,905 mm und eine Länge von 12 cm besitzt.

3. Vorrichtung zur Intubation der Tränenwege nach Anspruch 1, **dadurch gekennzeichnet, dass** das Segment mit mittlerem Durchmesser (26) einen Durchmesser von 1,143 mm und eine Länge von 15 cm besitzt.

4. Vorrichtung zur Intubation der Tränenwege nach Anspruch 1, **dadurch gekennzeichnet, dass** das distale Segment (28) einen Durchmesser von 0,762 mm und eine Länge von 7 cm besitzt.

5. Vorrichtung zur Intubation der Tränenwege nach Anspruch 1, **dadurch gekennzeichnet, dass** das feine Zentralsegment (24) einen Durchmesser von 0,762 mm und eine Länge von 20 mm besitzt.

6. Vorrichtung zur Intubation der Tränenwege nach Anspruch 1, **dadurch gekennzeichnet, dass** sie Übergangssegmente (32, 34, 36) zwischen dem Endsegment (22) und dem feinen Zentralsegment (24), zwischen dem feinen Zentralsegment und dem Segment mit mittlerem Durchmesser (26) und zwischen dem Segment mit mittlerem Durchmesser und dem distalen Segment (28) umfasst.

7. Vorrichtung zur Intubation der Tränenwege nach Anspruch 1, **dadurch gekennzeichnet, dass** das Endsegment (22) ein Licht (37) trägt, das sich an seinem freien Ende öffnet.

## Claims

1. An apparatus for intubation of the lacrimal duct that is intended to be inserted into an orifice formed by surgery between the lacrimal sac and the nasal cavity of the patient, the said apparatus being formed by a flexible, elongated element made of silicon (20), having, at one end, a terminal segment (22) having a larger diameter than the other parts of the said apparatus, this terminal segment (22) being connected to a segment (26) of average diameter having a smaller diameter than that of the terminal segment by a fine central segment (24), whereas the free end of the segment of average diameter (26) is extended by a distal segment (28) having a diameter less than that of the segment of average diameter (26) and the free end of which comprises an aperture (31).

2. An apparatus for intubation of the lacrimal duct according to Claim 1,
**characterised in that** the terminal segment (22) has a diameter of 1.905 mm and a length of 12 cm.

3. An apparatus for intubation of the lacrimal duct according to Claim 1,
**characterised in that** the segment of average diameter (26) has a diameter of 1.143 mm and a length of 15 cm.

4. An apparatus for intubation of the lacrimal duct according to Claim 1,
**characterised in that** the distal segment (28) has a diameter of 0.762 mm and a length of 7 cm.

5. An apparatus for intubation of the lacrimal duct according to Claim 1,
**characterised in that** the fine central segment (24) has a diameter of 0.762 mm and a length of 20 mm.

6. An apparatus for intubation of the lacrimal duct according to Claim 1,
**characterised in that** it comprises transitional segments (32, 34, 36) between the terminal segment (22) and the fine central segment (24), between the fine central segment and the segment of average diameter (26) and between the segment of average diameter and the distal segment (28).

7. An apparatus for intubation of the lacrimal duct according to Claim 1,
**characterised in that** the terminal segment (22) comprises an aperture (27) opening at its free end.
